# EUROPÄISCHE PATENTANMELDUNG

(11) **EP 2 628 478 A2**
(43) Veröffentlichungstag der Anmeldung: **21.08.2013**
(21) Anmeldenummer: 12191251.3
(22) Anmeldetag: 05.11.2012
(51) Int. Cl.: A61K 8/34, A61Q 19/00, A61K 8/37, A61K 8/49, A61K 8/97

(54) **Kosmetische Verwendung einer Kombination spezieller alpha,omega-Diole, einer speziellen Toco-Verbindung und einer Triglyzeridfraktion der Pflanze vom Typ Butyrospermum Parkii zum Schutz und/oder zur Pflege der Haut**

(30) Priorität: 20.12.2011 DE 102011089269
(71) Anmelder: Henkel AG&Co. KGAA, 40589 Düsseldorf (DE)
(72) Erfinder: Welß, Thomas, 40591 Düsseldorf (DE); Waldmann-Laue, Marianne, 40789 Monheim (DE); Elias, Kerstin, 51371 Leverkusen (DE); Giesen, Melanie, 47608 Geldern (DE)

(57) **Zusammenfassung**

Hautkosmetisches Mittel, enthaltend in einem kosmetisch akzeptablen Träger eine Wirkstoffkombination aus
- mindestens einer Verbindung der Formel (W1)

HO-CH₂(CH₂)ₙCH₂-OH (W1)

mit n gleich 2, 3, 4, 5 oder 6 (insbesondere n = 4),

und
- mindestens einer Tocol-Verbindung ausgewählt aus mindestens einer Verbindung der Formel (W2) (insbesondere α-Tocopherol) 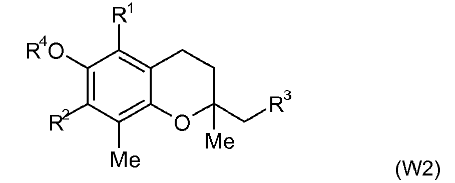 worin
R¹ und R² unabhängig voneinander für ein Wasserstoffatom oder eine Methylgruppe stehen, und
R³ für eine Formel (W2-a) oder Formel (W2-b) steht 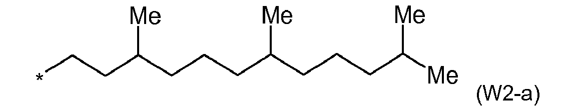 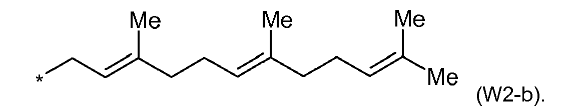 R⁴ für ein Wasserstoffatom oder eine (C₂ bis C₆)-Acylgruppe steht,

und
- eine Triglyceridfraktion, die aus einer Pflanze vom Typ *Butyrospermum Parkii* gewonnen wird, eignen sich besonders gut zur Regeneration der Hautbarriere und zur kosmetischen nichttherapeutischen Verwendung gegen Hauttrockenheit und zur Faltenreduktion.

## Beschreibung

Die Erfindung betrifft das technische Gebiet der kosmetischen Hautpflege. Insbesondere schlägt die technische Lehre der vorliegenden Erfindung die Verwendung einer Wirkstoffkombination aus alpha, omega-(C₄ bis C₈)-Diol, eine spezielle Tocol-Verbindung und einer Triglyzeridfraktion aus einer Pflanze vom Typ Butyrospermum Parkii in einem kosmetischen Mittel zur Hautbehandlung, insbesondere zum Schutz und/oder zur Pflege der Haut, vor.

Während man früher zwischen Mitteln zur Pflege des menschlichen Körpers und solchen zur Verschönerung seines Aussehens unterschied nach "Körperpflegemitteln" und "dekorativen Kosmetika", werden diese Produkte heute zusammengefasst definiert als "*kosmetische Mittel*" *oder* kosmetische Zusammensetzungen.

Angesichts der Allgemeinzugänglichkeit der kosmetischen Mittel und ihrer Anwendung am menschlichen Körper besteht zum Schutz des Verbrauchers in Deutschland und der Europäischen Union ein umfangreiches Regelwerk. Gesetzliche Grundlage in Deutschland ist das Lebensmittel- und Bedarfsgegenstände-Gesetz (LMBG), das kosmetische Mittel in § 4 wie folgt definiert:
"(1) kosmetische Mittel im Sinne dieses Gesetzes sind Stoffe oder Zubereitungen aus Stoffen, die dazu bestimmt sind, äußerlich am Menschen oder in seiner Mundhöhle zur Reinigung, Pflege oder zur Beeinflussung des Aussehens oder des Körpergeruchs oder zur Vermittlung von Geruchseindrücken angewendet zu werden, es sei denn, daß sie überwiegend dazu bestimmt sind, Krankheiten, Leiden, Körperschäden oder krankhafte Beschwerden zu lindern oder zu beseitigen." [§ 4 LMBG ist bisher nicht an die EU-Kosmetikrichtlinie angepasst, die Kosmetika sechs Funktionen (reinigen, parfümieren, Aussehen verändern, Körpergeruch beeinflussen, schützen und in gutem Zustand halten) zuspricht.]
"(2) Den kosmetischen Mitteln stehen Stoffe oder Zubereitungen aus Stoffen zur Reinigung oder Pflege von Zahnersatz gleich.
(3) Als kosmetische Mittel gelten nicht Stoffe oder Zubereitungen aus Stoffen, die zur Beeinflussung der Körperformen bestimmt sind."

Je nach Anwendungsgebiet unterscheidet man daher ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- u. Mundpflege (Zahn- und Mundpflegemittel, Gebißpflegemittel, Prothesenhaftmittel) und solche zur Haarpflege (Haarwaschmittel, Haarpflegemittel, Haarverfestigungsmittel, Haarverformungsmittel, Mittel zur Farbänderung).

Die Erfindung stellt kosmetische Mittel zur Hautpflege bereit. Die menschliche Haut übt als größtes Organ des Menschen zahlreiche lebenswichtige Funktionen aus. Mit ihrer Oberfläche von durchschnittlich etwa 2 m² beim erwachsenen Menschen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu. Die kosmetische Behandlung der Haut ist daher ein wichtiger Bestandteil der menschlichen Körperpflege. Ziel der Hautpflege im kosmetischen Sinn ist die natürliche Funktion der Haut als Barriere gegen Witterungs- und Umwelteinflüsse und gegen den Verlust von körpereigenen Stoffen (z.B. Wasser, natürliche Fette, Elektrolyte) zu stärken oder wiederherzustellen und den natürlichen Alterungsprozess zu verlangsamen, um der Haut ein junges und gesundes Aussehen zu verleihen.

Von besonderem Interesse sind daher kosmetische Wirkstoffe zur Behandlung der Haut, die pflegende, vor Alterserscheinungen schützende und revitalisierende Eigenschaften vermitteln. Präparate zur Hautpflege und zum Hautschutz, die eine ganze Reihe unterschiedlicher kosmetischer Wirkstoffe- oder Wirkstoffkomplexe enthalten, werden in großer Zahl und in vielen Zubereitungsformen angeboten.

Dennoch besteht nach wie vor das Bedürfnis den Stand der Technik durch neue kosmetische Wirkstoffe oder Wirkstoffkomplexe zu bereichen. Vor allem sind solche Wirkstoffe von großem Interesse, die einen nachhaltigen Schutz und/oder eine verbesserte Pflege der Haut ermöglichen. Die Stärkung der Barrierefunktion der Haut und die Steigerung des Hautzellmetabolismus zur Anregung der Verjüngung der Hautzellen fördert ein gesundes Aussehen der Haut. Die Haut ist weniger rau, weniger trocken und die Faltenbildung kann reduziert werden.

Die Barrierefunktion der Haut schützt die Haut vor dem Austrocknen. Bewirkt wird diese Barrierefunktion durch die Epidermis, welche als äußerste Schicht die eigentliche Schutzhülle gegenüber der Umwelt bildet. Die Epidermis ist ein stratifiziertes Gewebe, in dem die äußere Schicht, die Hornschicht (stratum corneum) den für die Barrierefunktion bedeutenden Teil darstellt. Sie wird im Kontakt mit der Umwelt abgenutzt und befindet deshalb sich in einem ständigen Erneuerungsprozess, wobei nach außen kontinuierlich feine Schuppen abgegeben und von innen verhorntes Zell- und Lipidmaterial nachproduziert wird.

Das heute in der Fachwelt anerkannte Hautmodell von Elias (P. M. Elias, Structure and Function of the Stratum Corneum Permeability Barrier, Drug Dev. Res. 13, 1988, 97 - 105) beschreibt die Hornschicht als Zwei-Komponenten-System, ähnlich einer Ziegelsteinmauer (Ziegelstein-Mörtel-Modell). In diesem Modell entsprechen die Hornzellen (Corneozyten) den Ziegelsteinen, die komplex zusammengesetzte Lipidmembran in den Interzellularräumen entspricht dem Mörtel. Dieses System stellt im Wesentlichen eine physikalische Barriere gegen hydrophile Substanzen dar, kann aber aufgrund seiner engen und mehrschichtigen Struktur gleichermaßen auch von lipophilen Substanzen nur schwer passiert werden. Die besondere Struktur der Hornschicht schützt einerseits die Haut und stabilisiert andererseits ihre eigene Flexibilität durch Bindung einer definierten Wassermenge.

Auch mechanische Belastungen, wie beispielsweise Druck-, Stoß- oder Scherkräfte, können in erstaunlichem Maße durch die Hornschicht allein oder im Verbund mit den tieferen Hautschichten abgefangen werden. Größere Druck-, Dreh- oder Scherkräfte werden über die Verzahnung der Epidermis mit dem Corium an tiefere Hautschichten weitergegeben. Die Regulation des Wasser-und Feuchtigkeitsgehaltes ist eine der wichtigsten Funktionen der epidermalen Lipidmembran. Allerdings hat sie nicht nur eine Barrierewirkung gegen externe chemische und physikalische Einflüsse, sondern trägt auch zum Zusammenhalt der Hornschicht bei. Bereits bei einer Reinigung der Haut mit Hilfe eines einfachen Wasserbads - ohne Zusatz von Tensiden - kommt es zunächst zu einer Quellung der Hornschicht der Haut. Gleichzeitig werden wasserlösliche Stoffe ab- bzw. ausgewaschen, wie z. B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Durch umweltbedingte Abnutzungsschäden bzw. Irritationen der Haut, Lichtschäden, Altershaut usw., ist der Schutzmechanismus an der Hautoberfläche gestört. Bei alter Haut beispielsweise erfolgt die regenerative Erneuerung verlangsamt, wobei insbesondere das Wasserbindungsvermögen der Hornschicht nachlässt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen.

Um die Haut bei ihrer natürlichen Regeneration zu unterstützen und ihre physiologische Funktion zu stärken, werden den topischen Präparaten in neuerer Zeit zunehmend Interzellularlipidmischungen, wie Ceramide oder Ceramidanaloga, zugesetzt, die von der Haut zum Wiederaufbau der natürlichen Barriere verwendet werden sollen. Allerdings handelt es sich bei diesen Lipiden zumeist um sehr teure Rohstoffe, die außerdem aufgrund ihres hohen Schmelzpunktes schwierig zu formulieren sind. Zudem ist ihre Wirkung begrenzt, da die topisch applizierten Lipide nur in geringem Umfang in die barriererelevanten Lipidmembranen im mittleren Stratum corneum penetrieren und nur zu einem geringen Teil in diese Strukturen integriert werden. Dem Stand der Technik mangelt es demnach an Zubereitungen, die die Barrierefunktion und die Hydratation der Hornschicht positiv beeinflussen und die physikalisch-chemischen Eigenschaften der Hornschicht und insbesondere der Lamellen aus Interzellularlipiden stärken bzw. sogar wiederherstellen.

Aufgabe der vorliegenden Erfindung war es, topische Zusammensetzungen zu entwickeln, die die Barrierefunktion der Haut, beispielsweise gegenüber dem transepidermalen Wasserverlust, stärken und eine Verjüngung der Hautzellen anregt. Eine weitere Aufgabe der vorliegenden Erfindung war es, topische Zusammensetzungen zu entwickeln, die die Barrierefunktion der Haut stärken. Eine weitere Aufgabe der vorliegenden Erfindung war es, kosten- und herstellungsoptimierte topische Zusammensetzungen zu entwickeln, die die Barrierefunktion der Haut stärken und eine Verjüngung der Hautzellen anregt.

Ein erster Gegenstand der vorliegenden Erfindung ist die nicht-therapeutische kosmetische Verwendung einer Wirkstoffkombination aus
- mindestens einer Verbindung der Formel (W1)

   HO-CH₂(CH₂)ₙCH₂-OH (W1)

   mit n gleich 2, 3, 4, 5 oder 6 (insbesondere n = 4),
   und
- mindestens einer Tocol-Verbindung ausgewählt aus mindestens einer Verbindung der Formel (W2) 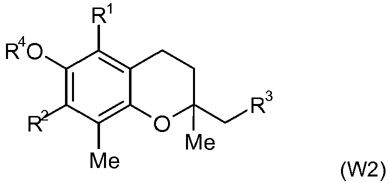 worin
   R¹ und R² unabhängig voneinander für ein Wasserstoffatom oder eine Methylgruppe stehen, und
   R³ für eine Formel (W2-a) oder Formel (W2-b) steht 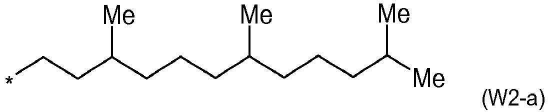 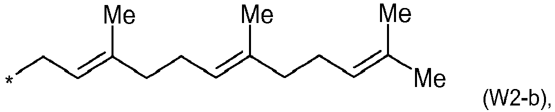 R⁴ für ein Wasserstoffatom oder eine (C₂ bis C₆)-Acylgruppe (insbesondere Acetyl) steht, und
- einer Triglyceridfraktion, die aus einer Pflanze vom Typ *Butyrospermum Parkii* gewonnen wird zur Regeneration der Hautbarrierefunktion. Ein weiterer Gegenstand der vorliegenden Erfindung ist die nicht-therapeutische kosmetische Verwendung der vorgenannten Wirkstoffkombination zur Beschleunigung der Regeneration der Hautbarrierefunktion. Ein weiterer Gegenstand der vorliegenden Erfindung ist die nicht-therapeutische kosmetische Verwendung der vorgenannten Wirkstoffkombination zur Verbesserung des Erscheinungsbildes trockener Haut. Ein weiterer Gegenstand der vorliegenden Erfindung ist die nicht-therapeutische kosmetische Verwendung der vorgenannten Wirkstoffkombination zur Erhöhung der Hautfeuchtigkeit. Daraus resultiert ein nicht-therapeutische kosmetische Verwendung der vorgenannten Wirkstoffkombination für die Hautstraffung. Ein weiterer Gegenstand der vorliegenden Erfindung ist die nicht-therapeutische kosmetische Verwendung der vorgenannten Wirkstoffkombination zur Erhöhung der Hautfeuchtigkeit und dadurch der Regeneration der Hautbarrierefunktion. Ein weiterer Gegenstand der vorliegenden Erfindung ist die nicht-therapeutische kosmetische Verwendung der vorgenannten Wirkstoffkombination zur Verjüngung der Hautzellen. Daraus resultiert ein nicht-therapeutische kosmetische Verwendung der vorgenannten Wirkstoffkombination für ein jüngeres Aussehen der Haut. Aus der Verjüngung der Hautzellen resultiert eine nicht-therapeutische kosmetische Verwendung der vorgenannten Wirkstoffkombination zur Faltenreduktion. Unter Faltenreduktion versteht sich im Sinne der Erfindung die Reduktion der Faltentiefe und/oder eine Reduktion der Faltenanzahl. Ein weiterer Gegenstand der vorliegenden Erfindung ist die nicht-therapeutische kosmetische Verwendung der vorgenannten Wirkstoffkombination zur Stimulierung der epidermalen Synthese von Wachstumsfaktoren, insbesondere des keratinozyten Wachstumsfaktors (KGF) und des Hepatozyten Wachstumsfaktors (HGF).

Eine mit dem Symbol * gekennzeichnete Bindung in Formeln dieser Anmeldung entspricht einer freien Valenz der entsprechenden Struktureinheit.

Zur Bestimmung der Effektivität der erfindungsgemäßen Wirkstoffkombination siehe Beispielsektion der vorliegenden Anmeldung (*vide infra*).

Erfindungsgemäß bevorzugt geeignete Tocol-Verbindungen sind die des zweiten Erfindungsgegenstandes (*vide infra*).

Die besagte Wirkstoffkombination wird üblicherweise zur einfacheren Dosierung und Applikation auf die Haut in einen kosmetischen Träger eingearbeitet unter Erhalt eines hautkosmetischen Mittels. Ein zweiter Gegenstand der Erfindung ist daher ein hautkosmetisches Mittel, enthaltend in einem kosmetisch akzeptablen Träger eine Wirkstoffkombination aus
- mindestens einer Verbindung der Formel (W1)

   HO-CH₂(CH₂)ₙCH₂-OH (W1)

   mit n gleich 2, 3, 4, 5 oder 6 (insbesondere n = 4),
   und
- mindestens einer Tocol-Verbindung ausgewählt aus mindestens einer Verbindung der Formel (W2) 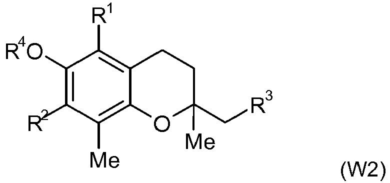 worin
   R¹ und R² unabhängig voneinander für ein Wasserstoffatom oder eine Methylgruppe stehen, und
   R³ für eine Formel (W2-a) oder Formel (W2-b) steht 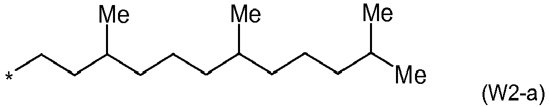 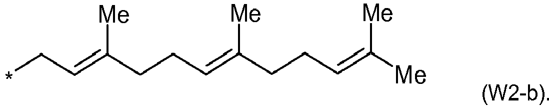 R⁴ für ein Wasserstoffatom oder eine (C₂ bis C₆)-Acylgruppe (insbesondere Acetyl) steht, und
- eine Triglyceridfraktion, die aus einer Pflanze vom Typ *Butyrospermum Parkii* gewonnen wird.

Erfindungsgemäß bevorzugt verwendete kosmetische Hautbehandlungsmittel enthalten die Verbindungen der obigen Formel (W1), insbesondere 1,6-Hexandiol, bevorzugt in einer Menge von 0,5 bis 10,0 Gew.-%, besonders bevorzugt von 4,0 bis 8,0 Gew.-%, jeweils bezogen auf das Gesamtgewicht des Mittels.

R⁴ der Formel (W2) steht bevorzugt für ein Wasserstoffatom.

Als "Tocol" bezeichnet man im allgemeinen die chemische Verbindung 2-Methyl-2-(4',8',12'-trimethyltridecyl)chroman-6-ol. Das erfindungsgemäße Mittel enthält zwingend mindestens eine der zuvor definierten Tocol-Verbindungen der Formel (W2). Die Tocol-Verbindungen sind chiral. Alle Stereoisomere der entsprechend genannten Verbindungen sind erfindungsgemäß. Bevorzugt sind die natürlich vorkommenden Stereoisomere oder synthetisch zugängliche Racemate.

Die Gruppe, aus der bevorzugt mindestens ein oder mehrere erfindungsgemäß verwendete Tocol-Verbindungen ausgewählt werden, gehorcht der Formel (W2-1) 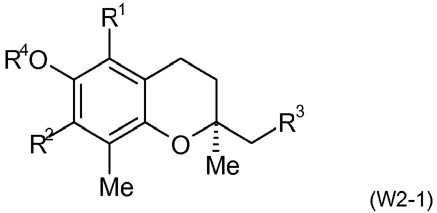 worin
R¹ und R² unabhängig voneinander für ein Wasserstoffatom oder eine Methylgruppe stehen, und R³ für eine Formel (W2-1a) oder Formel (W2-1 b) steht 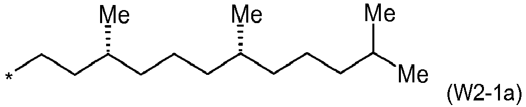 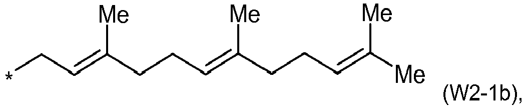 R⁴ für ein Wasserstoffatom oder eine (C₂ bis C₆)-Acylgruppe (insbesondere Acetyl) steht.

Erfindungsgemäß bevorzugte Mittel enthalten mindestens eine Tocol-Verbindung, die ausgewählt wird aus mindestens einer Verbindung der Gruppe, die gebildet wird aus α-Tocopherol (R¹=Me, R²=Me , R³=Formel (W2-1a), R⁴=H), β-Tocopherol (R¹=Me, R²=H, R³=Formel (W2-1a), R⁴=H), γ-Tocopherol (R¹=H, R²=Me, R³=Formel (W2-1a), R⁴=H), ö-Tocopherol (R¹=H, R²=H, R³=Formel (W2-1a), R⁴=H), α-Tocotrienol (R¹=Me, R²=Me R³=Formel (W2-1b), R⁴=H), β-Tocotrienol (R¹=Me, R²=H R³=Formel (W2-1 b), R⁴=H), γ-Tocotrienol (R¹=H, R²= Me R³=Formel (W2-1 b), R⁴=H) und δ-Tocotrienol (R¹=H, R²=H R³=Formel (W2-1b), R⁴=H), α-Tocopherolacetat (R¹=Me, R²=Me, R³=Formel (W2-1a), R⁴=*-COCH₃), β-Tocopherolacetat (R¹=Me, R²=H, R³=Formel (W2-1a), R⁴=*-COCH₃), γ-Tocopherolacetat (R¹=H, R²=Me, R³=Formel (W2-1a), R⁴=*-COCH₃), δ-Tocopherolacetat (R¹=H, R²=H, R³=Formel (W2-1a), R⁴=*-COCH₃), α-Tocotrienolacetat (R¹=Me, R²=Me R³=Formel (W2-1 b), R⁴=*-COCH₃), β-Tocotrienolacetat (R¹=Me, R²=H R³=Formel (W2-1 b), R⁴=*-COCH₃), γ-Tocotrienolacetat (R¹=H, R²=Me R³=Formel (W2-1 b), R⁴=*-COCH₃) und δ-Tocotrienolacetat (R¹=H, R²= H R³=Formel (W2-1b)), R⁴=*-COCH₃).

α-Tocopherol und/oder Tocopherolacetat sind erfindungsgemäß die bevorzugteste Tocol-Verbindung. Am bevorzugtesten ist α-Tocopherol.

Erfindungsgemäß bevorzugte kosmetische Hautbehandlungsmittel enthalten die Tocol-Verbindungen der Formel (W2), bevorzugt der Formel (W2-1), ganz besonders bevorzugt α-Tocopherol, bevorzugt in einer Menge von 0,01 bis 1,0 Gew.-%, besonders bevorzugt von 0,02 bis 0,1 Gew.-%, jeweils bezogen auf das Gesamtgewicht des Mittels.

Als dritte zwingende Komponente enthält das erfindungsgemäße Mittel eine Triglyceridfraktion, die aus einer Pflanze vom Typ *Butyrospermum Parkii* gewonnen wird

Eine erfindungsgemäße Pflanze vom Typ *Butyrospermum Parkii* trägt den wissenschaftlichen Namen *Vitellaria paradoxa* und wird umgangssprachlich auch Karitebaum oder Sheanussbaum genannt. Diese Pflanze stammt aus der Familie der Sapotengewächse (Sapotaceae) und kommt in den sudanischen Savannen im tropischen Afrika zwischen Senegal und Uganda in zwei Unterarten vor. Die Früchte dieser bis zu 20m hoch werdenden Bäume sind botanisch gesehen Beeren und enthalten etwa 4cm große Samen, die als Sheanuss bezeichnet werden und einen Fettanteil von ca. 50% aufweisen. Das Beerenfleisch kann von Mensch und Tier als Obst gegessen werden.

Die Triglyceridfraktion (Fettfraktion) wird aus den Samen üblicherweise durch mechanische oder chemische Verfahren gewonnen.

Im Rahmen des mechanischen Verfahrens werden die Samen gründlich mechanisch zerkleinert (vorzugsweise gestampft), die resultierende Masse in Wasser gekocht, und anschließend wird das oben schwimmende Fett abgeschöpft. Das so gewonnene Rohfett ist graugelb und weist einen eigenartigen Geruch auf. In einem zweiten Schritt wird das so gewonnene Rohfett häufig raffiniert, so dass ein butterartiges, zähes und geruchsloses Fett erhalten wird.

Ein übliches chemisches Verfahren ist die Extraktion der zerkleinerten Samen mit Hexan. Mit Hilfe dieses Verfahrens können höhere Ausbeuten der Fettfraktion erhalten werden. Allerdings werden die unverseifbaren Bestandteile deutlich schlechter extrahiert, und das resultierende Fett weist einen geringen Anteil an unverseifbaren Bestandteilen auf.

Die Triglyceridfraktion dieser Pflanzenart zeichnet sich durch ihren hohen Ölsäure und Stearinsäuregehalt aus.

Erfindungsgemäß bevorzugt sind Trigylceridfraktionen, die 45-55Gew.-% Ölsäure, 30-45Gew.-% Stearinsäure, 2-8Gew.-% Palmitinsäure und 2-8Gew.-% Linolsäure enthalten, besonders bevorzugt sind Triglyceridfraktionen, die 49-50Gew.-% Ölsäure, 36-42 Gew.-% Stearinsäure, 5-6 Gew.-% Palmitinsäure und 4-5 Gew.-% Linolsäure enthalten.

Im Rahmen einer bevorzugten Ausführungsform enthalten die erfindungsgemäßen Mittel sowohl eine Triglyceridfraktion als auch eine unverseifbare Fraktion, die beide aus einer Pflanze vom Typ *Butyrospermum Parkii* gewonnen werden. Im Rahmen dieser Ausführungsform hat es sich als vorteilhaft erwiesen, wenn der Anteil der unverseifbaren Fraktion bezogen auf den gesamte Bestandteil, der aus der Pflanze vom Typ *Butyrospermum Parkii* gewonnen wird, mindestens 5Gew.-%, vorzugsweise mindestens 9 Gew.-%, beträgt.

Als unverseifbare Bestandteile enthalten die Sheanüsse insbesondere Triterpenalkohole, Phytosterole, Latex, Vitamine sowie Allantoin. Im Gegensatz zu vielen anderen Fett-haltigen Pflanzen, wie beispielsweise Sesam oder Oliven, weisen die Pflanzen vom Typ *Butyrospermum Parkii* einen relativ hohen Anteil an unverseifbaren Bestanteilen auf, die die Eigenschaften der gewonnenen Fettfraktion wesentlich prägen.

Die Triglyceridfraktion ggf gemeinsam mit der unverseifbaren Fraktion, die jeweils aus Pflanzen vom Typ *Butyrospermum Parkii* gewonnen werden, sind erfindungsgemäß vorzugsweise in Mengen von 0,5 bis 10,0 Gew.-%, insbesondere von 1,0 bis 5,0 Gew.-%, jeweils bezogen auf das Gesamtgewicht des erfindungsgemäßen Mittels, in den erfindungsgemäßen Mitteln enthalten.

Es hat sich als erfindungsgemäß vorteilhaft erwiesen, zur Steigerung der erfindungsgemäßen Effekte in den erfindungsgemäßen Mitteln zusätzlich mindestens ein Oligoglykosid der Formel (W3) einzusetzen

R¹-O-[G]ₚ (W3)

worin
- R¹: für einen Alkyl- und/oder Alkenylrest mit 4 bis 22 Kohlenstoffatomen,
- G: für eine Glykosideinheit, die sich von einem Zucker mit 5 oder 6 Kohlenstoffatomen ableitet, und
- p: für Zahlen von 1 bis 10 steht.

Die Oligoglykoside der Formel (W3) können sich gemäß ihrer Glykosideinheit G von Aldosen bzw. Ketosen mit 5 oder 6 Kohlenstoffatomen, vorzugsweise der Glucose ableiten. Die bevorzugten Oligoglykoside der Formel (W3) sind somit Alkyl- und/oder Alkenyloligoglucoside.

Die Indexzahl p in der allgemeinen Formel (W3) gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- - und/oder Alkenyloligoglykoside mit einem mittleren Oligomerisierungsgrad p von 1,1 bis 3,0 eingesetzt. Aus anwendungstechnischer Sicht sind solche Oligoglykoside der Formel (W3) bevorzugt, deren Oligomerisierungsgrad kleiner als 1,7 ist und insbesondere zwischen 1,2 und 1,4 liegt.

Der Alkyl- bzw. Alkenylrest R¹ leitet sich bevorzugt von primären Alkoholen mit 12 bis 22, vorzugsweise 18 bis 22 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können.

Besonders bevorzugt steht in Formel (W3) R¹ für eine (C₁₈-C₂₂)-Alkylgruppe und G leitet sich von Glucose ab.

Ganz besonders bevorzugt wird das Oligoglykosid aus Verbindungen der Formel (W3) ausgewählt, in denen R¹ für eine Alkylgruppe mit 20 Kohlenstoffatomen und G sich von Glucose ableitet. Das Oligoglykosid dieser Struktur trägt die INCI-Bezeichnung Arachidyl Glucoside.

Die Oligoglykoside der Formel (W3) sind erfindungsgemäß vorzugsweise in Mengen von 0,1 bis 10,0 Gew.-%, insbesondere von 0,5 bis 3,0 Gew.-%, jeweils bezogen auf das Gesamtgewicht des erfindungsgemäßen Mittels, in den erfindungsgemäßen Mitteln enthalten.

Die erfindungsgemäße Wirkstoffkombination ist in einen kosmetisch akzeptablen Träger eingearbeitet. Als solche kosmetisch akzeptablen Träger eignen sich bevorzugt für die erfindungsgemäßen kosmetischen Hautbehandlungsmittel flüssige, fließfähige oder feste Öl-in-Wasser-Emulsion, Wasser-in-Öl-Emulsionen, Mehrfach-Emulsionen, insbesondere einer Öl-in-Wasser-in-Öl- oder Wasser-in-Öl-in-Wasser-Emulsion, Makroemulsionen, Miniemulsionen, Mikroemulsionen, PIT-Emulsionen, Nanoemulsionen, Pickering-Emulsionen, Hydrodispersionen eines Hydrogels, eines Lipogels, eine ein- oder mehrphasigen Lösung, ein Schaum, eines Puder. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer erfindungsgemäß besonders bevorzugt verwendeten Ausführungsform liegen die Hautbehandlungsmittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen. In der Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn - Salze.

Die erfindungsgemäß verwendeten Hautbehandlungsmittel enthalten die Emulgatoren bevorzugt in Mengen von 0,1 bis 25 Gew.-%, insbesondere 0,5 - 15 Gew.-%, bezogen auf das gesamte Mittel. In einer besonders bevorzugt verwendeten Ausführungsform ist mindestens ein nichtionischer Emulgator mit einem HLB-Wert von 8 und darunter enthalten. Derart geeignete Emulgatoren sind beispielsweise Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.

Weitere geeignete Zusatzstoffe sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropan-sulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS, Simulgel^{®} EG und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Die erfindungsgemäß verwendeten Hautbehandlungsmittel enthalten bevorzugt Fettstoffe, die ausgewählt sind aus Ölen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt 0,1 - 50 Gew.%, bevorzugt 0,1 - 20 Gew.% und besonders bevorzugt 0,1 - 15 Gew.%, jeweils bezogen auf das gesamte Hautbehandlungsmittel.

Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Bevorzugte hautkosmetische Mittel enthalten in einem kosmetisch akzeptablen Träger eine Wirkstoffkombination aus - jeweils bezogen auf das Gesamtgewicht des hautkosmetischen Mittels -
- 0,5 bis 10,0 Gew.-%, insbesondere 4,0 bis 8,0 Gew.-% mindestens einer Verbindung der Formel (W1)

   HO-CH₂(CH₂)ₙCH₂-OH (W1)

   mit n gleich 2, 3, 4, 5 oder 6 (insbesondere n = 4),
   und
- 0,01 bis 1,0 Gew.-%, insbesondere 0,02 bis 0,1 Gew.-%, mindestens einer Tocol-Verbindung ausgewählt aus mindestens einer Verbindung der Formel (W2) (insbesondere α-Tocopherol) 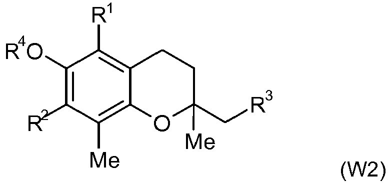 worin
   R¹ und R² unabhängig voneinander für ein Wasserstoffatom oder eine Methylgruppe stehen, und
   R³ für eine Formel (W2-a) oder Formel (W2-b) steht 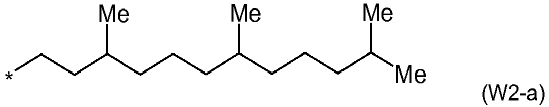 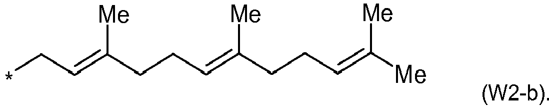 R⁴ für ein Wasserstoffatom oder eine (C₂ bis C₆)-Acylgruppe steht,
   und
- 0,5 bis 10,0 Gew.-%, insbesondere 4,0 bis 8,0 Gew.-%, einer Triglyceridfraktion, die aus einer Pflanze vom Typ *Butyrospermum Parkii* gewonnen wird,
   und gegebenenfalls
- 0,1 bis 10,0 Gew.-%, insbesondere 0,5 bis 3,0 Gew.-% zusätzlich mindestens eines Oligoglykosids der Formel (W3)

   R¹-O-[G]ₚ (W3)

   worin
   - R¹: für einen Alkyl- und/oder Alkenylrest mit 4 bis 22 Kohlenstoffatomen,
   - G: für eine Glykosideinheit, die sich von einem Zucker mit 5 oder 6 Kohlenstoffatomen ableitet, und
   - p: für Zahlen von 1 bis 10 steht (insbesondere Arachidyl Glucoside).

### Beispiele

### 1.0 Wirknachweise

Es wurde folgende Zusammensetzung E1 als erfindungsgemäße Zusammensetzung hergestellt:

| Rohstoff | Gew.-% |
|---|---|
| 1,6-Hexandiol | 6,00 |
| Tocopherol | 0,03 |
| Triglyzeride aus *Butyrospermum Parkii* (Shea Butter) | 2,00 |
| Arachidyl Glucosid | 0,75 |
| Caprylic/Capric Triglyceride | 5,00 |
| Glycerin | 4,97 |
| Behenyl Alkohol | 4,50 |
| Dicaprylyl Carbonat | 3,00 |
| Arachidyl Alkohol | 2,75 |
| Cyclomethicone | 2,12 |
| 1,2-Propylenglycol | 2,00 |
| Cetearyl Alkohol | 1,00 |
| Carbomer | 0,40 |
| Hydroxyethyl Acrylate/Sodium Acryloyldimethyl | 0,375 |
| Taurate Copolymer | |
| 2-Phenoxyethanol | 0,375 |
| Dimethiconol | 0,351 |
| Parfum | 0,30 |
| Squalane | 0,25 |
| Ethylparaben | 0,06 |
| Methylparaben | 0,06 |
| Polysorbate 60 | 0,05 |
| Natriumhydroxid | 0,05 |
| Wasser | ad 100 |

### i) Steigerung des Hautzellmetabolismus

Zur Bestimmung der Steigerung des Hautzellmetabolismus wurden Ganzhautmodelle, ein Gewebsmodell welches den dermalen und den epidermalen Teil der Haut repräsentiert, mit den beiden zu vergleichenden Formulierungen behandelt und die Menge an Adenosin Triphosphat (ATP) im Gewebe bestimmt. Es je Testreihe wurden 4 Experimente durchgeführt und aus den Messergebnissen das arithmetische Mittel gebildet. Eine Testreihe wurde ohne die Applikation des Mittels E1 durchgeführt (Referenz) und eine weitere unter Applikation des Mittels E1. Zur relativen Angabe der ATP-Synthesestimulanz wurde die Referenz auf 100% berechnet.

| | Mittelwert [%] | Standardabweichung |
|---|---|---|
| unbehandelt (Referenz) | 100 | 14,44 |
| mit erfindungsgemäßem Mittel behandelt | 173 | 48,76 |

Die erfindungsgemäße Rezeptur führte zu einer signifikanten Steigerung der ATP-Synthese. Dies führt bei gesunder Haut zu einer Stimulierung der Synthese von Wachstumsfaktoren, zur Stimulierung der Zellteilung und zur Aktivierung der Barrierefunktion.

### ii) Freisetzung von Wachstumsfaktoren aus Co-Kulturen.

Bei einem Co-Kultursystem werden die Zellen der Hautmatrix, die Fibroblasten, als Einschichtkultur auf Kulturplatten gezüchtet. Ab einer bestimmten Bewuchsdichte werden Epidermismodell zur Kultur gegeben und, nach Akklimatisierung, die zu untersuchende Formel auf die Oberfläche der Epidermismodelle aufgetragen. 24h Stunden nach Behandlung werden die Mengen an freigesetztem Wachstumsfaktor aus dem Kulturmedium heraus quantifiziert und gegen die unbehandelte Kontrolle als Referenz berechnet.

### Freisetzung des Keratinozyten Wachstumsfaktors (KGF):

| | Mittelwert [%] | Standardabweichung |
|---|---|---|
| unbehandelt (Referenz) | 100 | 19 |
| mit erfindungsgemäßem Mittel behandelt | 150 | 19 |

### Freisetzung des Hepatozyten Wachstumsfaktors (HGF):

| | Mittelwert [%] | Standardabweichung |
|---|---|---|
| unbehandelt (Referenz) | 100 | 6 |
| mit erfindungsgemäßem Mittel behandelt | 197 | 9 |

### iii) Optimierung der Hautdurchfeuchtung und Barrierestruktur durch Stimulierung von Aquaporine-3

Die Steigerung der Aquaporin-3 Mengen in der Haut wurde nach Behandlung von Ganzhautmodellen (siehe oben Punkt i)) anhand von üblichen Immunhistologischen Färbungen mikroskopisch nachgewiesen.

Durch Vergleich der Fluoreszensintensität zwischen behandelten und unbehandelten Modellen, konnte der Fachmann eine deutliche Steigerung der Aquaporin-3 Menge in den Ganzhautmodellen nachweisen, die mit der Formel E1 behandelten worden waren. Dies ist ein deutlicher Hinweis darauf, dass vermehrt Aquaporine-3 Proteine in der Haut vorliegen. Dadurch kann die Haut besser durchfeuchtet werden, erscheint praller, glatter und gesünder.

### 2.0 Beispielrezepturen

### 2.1: Nachtcreme

| Rohstoff | [Gew.-%] |
|---|---|
| 1,6-Hexanediol | 6,00 |
| Tocopheryl Acetate | 0,50 |
| Triglyceridfraktion aus *Butyrospermum Parkii* (Shea Butter) | 1,00 |
| Taurin (2-Aminoethansulfonsäure) | 1,00 |
| Hydrolyzed Yeast Protein | 0,35 |
| Rosa Damascena Flower Water | 15,00 |
| Caprylic/Capric Triglyceride | 8,00 |
| Glycerin | 5,00 |
| Laurus Nobilis Leaf/Stem Water | 5,00 |
| Argania Spinosa Kernel Oil | 3,00 |
| Dicaprylyl Carbonate | 3,00 |
| Arachidyl Alcohol | 2,750000 |
| Behenyl Alcohol | 2,500000 |
| Betaine | 2,000000 |
| Carthamus Tinctorius Seed Oil | 2,000000 |
| Cocoglycerides | 1,000000 |
| Glyceryl Stearate | 1,000000 |
| Panthenol | 0,750000 |
| Dimethylsilanol Hyaluronate | 0,012000 |
| Echium Plantagineum Seed Oil | 0,499500 |
| Ectoin | 0,100000 |
| Bisabolol | 0,425000 |
| Allantoin | 0,200000 |
| Copernicia Cerifera Cera | 0,500000 |
| Magnesium Chlorid | 0,043500 |
| Sorbitan Oleate | 0,015000 |
| Aluminum Starch Octenylsuccinate | 0,930000 |
| Arachidyl Glucoside | 0,750000 |
| Cetearyl Alcohol | 0,500000 |
| Sodium Carbomer | 0,340000 |
| Bis-Ethylhexyl Hydroxydimethoxy Benzylmalonate | 0,400000 |
| Sodium Acrylate/Sodium Acryloyldimethyl Taurate | 0,187500 |
| Copolymer | |
| Polyisobutene | 0,137500 |
| Butylene Glycol | 0,087500 |
| Pentylene Glycol | 0,037500 |
| Caprylyl/Capryl Glucoside | 0,012500 |
| Pantolactone | 0,010000 |
| 2-Phenoxyethanol | 0,529500 |
| (2-Ethylhexyl)glycerin | 0,199600 |
| Natrium Benzoate | 0,062800 |
| Kalium Sorbat | 0,026400 |
| Wasser | ad 100 |

### 2.2 Feuchtigkeitsspendende Tagescreme

| Rohstoff | [Gew.-%] |
|---|---|
| 1,6-Hexanediol | 6,00 |
| Tocopherylacetat | 0,50 |
| Triglyceridfraktion aus *Butyrospermum Parkii* (Shea Butter) | 1,00 |
| Taurin (2-Aminoethansulfonsäure) | 1,00 |
| Rosa Damascena Flower Water | 15,00 |
| Caprylic/Capric Triglycerid | 8,00 |
| Glycerin | 4,95 |
| Laurus Nobilis Leaf/Stem Water | 5,00 |
| Argania Spinosa Kernel Oil | 3,00 |
| Arachidyl Alcohol | 2,75 |
| Behenyl Alcohol | 2,50 |
| Betaine | 2,00 |
| Carthamus Tinctorius Seed Oil | 2,00 |
| Cetearyl Isononanoate | 2,00 |
| Cocoglycerides | 1,00 |
| Glyceryl Stearate | 1,00 |
| Panthenol | 0,75 |
| Dimethylsilanol Hyaluronate | 0,012 |
| Echium Plantagineum Seed Oil | 0,50 |
| Ectoin | 0,10 |
| Bisabolol | 0,40 |
| Allantoin | 0,20 |
| Algae Extract | 0,022 |
| Magnesium Chlorid | 0,05 |
| Sorbitan Oleate | 0,015 |
| Aluminum Starch Octenylsuccinate | 0,90 |
| Arachidyl Glucoside | 0,75 |
| Cetearyl Alcohol | 0,50 |
| Sodium Carbomer | 0,32 |
| Bis-Ethylhexyl Hydroxydimethoxy Benzylmalonate | 0,40 |
| Sodium Acrylate/Sodium Acryloyldimethyl Taurate Copolymer | 0,20 |
| Polyisobutene | 0,15 |
| Butylene Glycol | 0,09 |
| Pentylene Glycol | 0,04 |
| Caprylyl/Capryl Glucoside | 0,013 |
| Pantolactone | 0,010 |
| Phenoxyethanol | 0,50 |
| (2-Ethylhexyl)glycerin | 0,20 |
| Natrium Benzoat | 0,06 |
| Kalium Sorbat | 0,02 |
| Wasser | ad 100 |

### 2.3 Antifalten-Creme

| Rohstoff | [Gew.-%] |
|---|---|
| 1,6-Hexandiol | 6,00 |
| Tocopherol | 0,002 |
| Tocopheryl Acetate | 0,50 |
| Triglyceridfraktion aus *Butyrospermum Parkii* (Shea Butter) | 1,00 |
| Taurin (2-Aminoethansulfonsäure) | 1,00 |
| Rosa Damascena Flower Water | 15,00 |
| Caprylic/Capric Triglyceride | 8,00 |
| Glycerin | 6,60 |
| Laurus Nobilis Leaf/Stem Water | 5,00 |
| Argania Spinosa Kernel Oil | 3,00 |
| Dicaprylyl Carbonate | 3,00 |
| Arachidyl Alcohol | 2,75 |
| Behenyl Alcohol | 2,50 |
| Betaine | 2,00 |
| Carthamus Tinctorius (Safflower) Seed Oil | 2,00 |
| Cocoglycerides | 1,00 |
| Glyceryl Stearate | 1,00 |
| Panthenol | 0,75 |
| Dimethylsilanol Hyaluronate | 0,012 |
| Echium Plantagineum Seed Oil | 0,50 |
| Ectoin | 0,10 |
| Bisabolol | 0,42 |
| Allantoin | 0,20 |
| Algae Extract | 0,022 |
| Palmitoyl Oligopeptide | 0,00033 |
| Palmitoyl Tetrapeptide-7 | 0,00015 |
| Copernicia Cerifera (Carnauba) Wax | 0,50 |
| Magnesium Chlorid | 0,043 |
| Natrium Lactate | 0,015 |
| Sorbitan Oleate | 0,015 |
| Aluminum Starch Octenylsuccinate | 1,40 |
| Arachidyl Glucoside | 0,75 |
| Cetearyl Alcohol | 0,50 |
| Sodium Carbomer | 0,46 |
| Bis-Ethylhexyl Hydroxydimethoxy Benzylmalonate | 0,40 |
| Sodium Acrylate/Sodium Acryloyldimethyl Taurate Copolymer | 0,18 |
| Polyisobutene | 0,13 |
| Butylene Glycol | 0,65 |
| Pentylene Glycol | 0,037 |
| Coco-Glucoside | 0,015 |
| Caprylyl/Capryl Glucoside | 0,012 |
| Pantolactone | 0,010 |
| 2-Phenoxyethanol | 0,25 |
| (2-Ethylhexyl)glycerin | 0,20 |
| Natrium Benzoat | 0,06 |
| Kalium Sorbat | 0,02 |
| Wasser | ad 100 |

## Patentansprüche

1. Nicht-therapeutische kosmetische Verwendung einer Wirkstoffkombination aus
- mindestens einer Verbindung der Formel (W1)
HO-CH₂(CH₂)ₙCH₂-OH (W1)
mit n gleich 2, 3, 4, 5 oder 6 (insbesondere n = 4),
und
- mindestens einer Tocol-Verbindung ausgewählt aus mindestens einer Verbindung der Formel (W2) 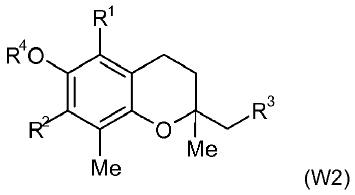 worin
R¹ und R² unabhängig voneinander für ein Wasserstoffatom oder eine Methylgruppe stehen, und
R³ für eine Formel (W2-a) oder Formel (W2-b) steht 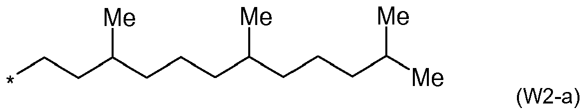 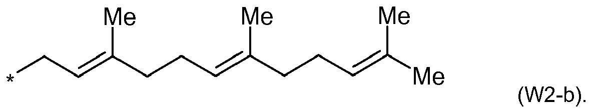 R⁴ für ein Wasserstoffatom oder eine (C₂ bis C₆)-Acylgruppe steht,
und
- einer Triglyceridfraktion, die aus einer Pflanze vom Typ *Butyrospermum Parkii* gewonnen wird,
zur Regeneration der Hautbarrierefunktion.

2. Nicht-therapeutische kosmetische Verwendung einer Wirkstoffkombination nach Anspruch 1 zur Reduktion von trockener Haut.

3. Nicht-therapeutische kosmetische Verwendung einer Wirkstoffkombination nach Anspruch 1 zur Erhöhung der Hautfeuchtigkeit.

4. Nicht-therapeutische kosmetische der Wirkstoffkombination nach Anspruch 1 für die Hautstraffung.

5. Nicht-therapeutische kosmetische Verwendung Wirkstoffkombination nach Anspruch 1 zur Faltenreduktion.

6. Hautkosmetisches Mittel, enthaltend in einem kosmetisch akzeptablen Träger eine Wirkstoffkombination aus
- mindestens einer Verbindung der Formel (W1)
HO-CH₂(CH₂)ₙCH₂-OH (W1)
mit n gleich 2, 3, 4, 5 oder 6 (insbesondere n = 4),
und
- mindestens einer Tocol-Verbindung ausgewählt aus mindestens einer Verbindung der Formel (W2) (insbesondere α-Tocopherol) 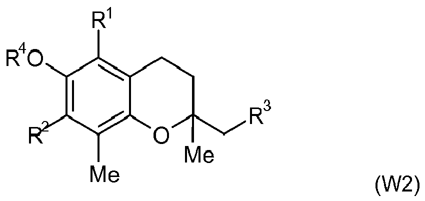 worin
R¹ und R² unabhängig voneinander für ein Wasserstoffatom oder eine Methylgruppe stehen, und
R³ für eine Formel (W2-a) oder Formel (W2-b) steht 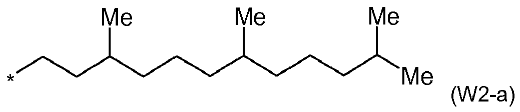 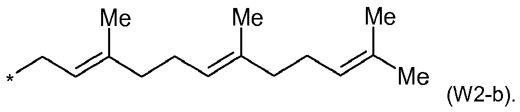 R⁴ für ein Wasserstoffatom oder eine (C₂ bis C₆)-Acylgruppe steht,
und
- eine Triglyceridfraktion, die aus einer Pflanze vom Typ *Butyrospermum Parkii* gewonnen wird.

7. Hautkosmetisches Mittel nach Anspruch 6, **dadurch gekennzeichnet, dass** es die Verbindungen der obigen Formel (W1), insbesondere 1,6-Hexandiol, in einer Menge von 0,5 bis 10,0 Gew.-%, bevorzugt von 4,0 bis 8,0 Gew.-%, jeweils bezogen auf das Gesamtgewicht des Mittels, enthält.

8. Hautkosmetisches Mittel nach einem der Ansprüche 6 oder 7, **dadurch gekennzeichnet, dass** es die Tocol-Verbindungen der Formel (W2), bevorzugt α-Tocopherol, in einer Menge von 0,01 bis 1,0 Gew.-%, bevorzugt von 0,02 bis 0,1 Gew.-%, jeweils bezogen auf das Gesamtgewicht des Mittels, enthält.

9. Hautkosmetisches Mittel nach einem der Ansprüche 6 bis 8, **dadurch gekennzeichnet, dass** es sowohl eine Triglyceridfraktion als auch eine unverseifbare Fraktion, die beide aus einer Pflanze vom Typ *Butyrospermum Parkii* gewonnen werden, enthält.

10. Hautkosmetisches Mittel nach einem der Ansprüche 6 bis 9, **dadurch gekennzeichnet, dass** es die Triglyceridfraktion ggf gemeinsam mit der unverseifbaren Fraktion, die beide jeweils aus Pflanzen vom Typ *Butyrospermum Parkii* gewonnen werden, in Mengen von 0,5 bis 10,0 Gew.-%, insbesondere von 1,0 bis 5,0 Gew.-%, jeweils bezogen auf das Gesamtgewicht des erfindungsgemäßen Mittels, enthält.

11. Hautkosmetisches Mittel nach einem der Ansprüche 6 bis 10, **dadurch gekennzeichnet, dass** es zusätzlich mindestens ein Oligoglykosid der Formel (W3) enthält
R¹-O-[G]ₚ (W3)
worin
R¹ für einen Alkyl- und/oder Alkenylrest mit 4 bis 22 Kohlenstoffatomen,
G für eine Glykosideinheit, die sich von einem Zucker mit 5 oder 6 Kohlenstoffatomen ableitet, und
p für Zahlen von 1 bis 10 steht.

12. Hautkosmetisches Mittel nach Anspruch 11, **dadurch gekennzeichnet, dass** es die Oligoglykoside der Formel (W3) in einer Menge von 0,1 bis 10,0 Gew.-%, insbesondere von 0,5 bis 3,0 Gew.-%, jeweils bezogen auf das Gesamtgewicht des erfindungsgemäßen Mittels, enthält.
